# EUROPEAN PATENT APPLICATION

(11) **EP 1 356 840 A1**
(43) Date of publication of application: **29.10.2003**
(21) Application number: 03003555.4
(22) Date of filing: 17.02.2003
(51) Int. Cl.: A61M 16/00

(54) **Anaesthetic filter arrangement**

(30) Priority: 23.04.2002 SE 0201213
(71) Applicant: Siemens-Elema AB, 171 95 Solna 1 (SE)
(72) Inventor: Ahlmén, Christer, 19251 Sollentuna (SE); Ambrus, Michael, 19271 Sollentuna (SE); Loncar, Mario, 17839 Ekerö (SE)

(57) **Abstract**

An anaesthetic filter arrangement for the reflection of unused anaesthetic agent in expiration gas back towards a patient comprises a filter housing (2) having an interior portion (8) arranged to provide an internal gas flow path for an inspiration gas and for an expiration gas passing alternately and in opposite directions through the housing (2) and a filter element (10) disposed in gas communication with the interior portion (8) for sorption of anaesthetic agent from the expiration gas and subsequent desorption of the anaesthetic agent into the inspiration gas. The arrangement further comprises thermal regulating means (12,18a,18b) operable to reduce thermal energy at the filter element (10) by cooling one or both incoming gas and the filter element (10) and thereby vary one or both the sorption and the desorption of the anaesthetic agent.

## Description

The present invention relates to an anaesthetic filter arrangement and in particular to a filter arrangement for the re-use of anaesthetics in inhalation anaesthesia.

Filter arrangements for the re-use of gaseous anaesthetics are well known and are described in, for example US 5,044,361 and US 5,471,979. These filter arrangements (so-called anaesthetic reflectors), generally comprise a filter housing in which there are provided openings delimiting a gas flow path through the interior of the housing. Disposed within the gas flow path is a filter element of an adsorption material for the alternate adsorption and desorption of gaseous anaesthetic from and into gas passing along the flow path. These filter elements are placed within gas flow circuits of anaesthetic ventilator systems so that anaesthetic rich expiration gas which is exhaled by a patient into the gas flow circuit during an expiration phase passes through the filter element along the flow path in one flow direction and so that inspiration gas in the gas flow circuit which is to be supplied to the patient during an inspiration phase passes through the filter element along the flow path, usually but not necessarily, in the opposite flow direction. The filter element adsorbs gaseous anaesthetic from the expiration gas then desorbs this adsorbed gaseous anaesthetic into the inspiration gas of the succeeding inspiration phase.

Such filter arrangements suffer from the disadvantage that their retention properties for the gaseous anaesthetic are fixed, dependent on the adsorption and desorption characteristics of the filter element material. Adsorption and desorption of gaseous anaesthetic to and from the filter element is then largely controlled by varying the flow of and to a lesser extent the concentration of gaseous anaesthetic in gas passing along the gas flow path, through the filter element. However, varying these gas flow and concentration parameters may have undesirable effects on the ventilation of a patient who is connected to an anaesthetic ventilator system in which the filter arrangement is disposed.

According to a first aspect of the present invention there is provided an anaesthetic filter arrangement as disclosed in and characterised by the present claim 1. The retention of gaseous anaesthetic by the filter element is thus varied by configuring the thermal regulating means to provide a reduced thermal energy at the element, for example by cooling incoming gas or by directly cooling the element, to thereby vary the adsorption and/or desorption of gaseous anaesthetic.

Usefully the thermal regulating means may be adapted to provide the cooling effect in synchronism with an expiration phase of a patient breathing cycle to thereby increase adsorption of the gaseous anaesthetic.

Additionally, the thermal regulating means may be further configured to provide an increased thermal energy at the filter element. The retention of gaseous anaesthetic by the filter element is thus varied by additionally providing thermal energy at the element, for example by warming incoming gas or by directly warming the element, to thereby vary the adsorption and/or desorption of gaseous anaesthetic.

Usefully the provision of the thermal regulating means may be adapted to provide the thermal energy in synchronism with an inspiration phase of a patient breathing cycle to thereby increase desorption of the gaseous anaesthetic into breathing gas for supply to a patient.

Preferably, the thermal regulating means can comprise one or more thermo-electric (Peltier effect) elements, having a cooling surface located in intimate thermal contact with the filter element or with incoming expiration gas and, optionally, having a heating surface located in intimate thermal contact with the filter element or with incoming inspiration gas.

Exemplary embodiments of the present invention will now be described with reference to the drawings of the accompanying figures, of which:
Fig. 1 shows an embodiment of a filter arrangement according to the present invention;
Fig. 2a shows an internal portion of a thermal regulating means suitable for use in the arrangement of Fig. 1;
Fig. 2b shows an external view of the thermal regulating means of Fig. 2a;

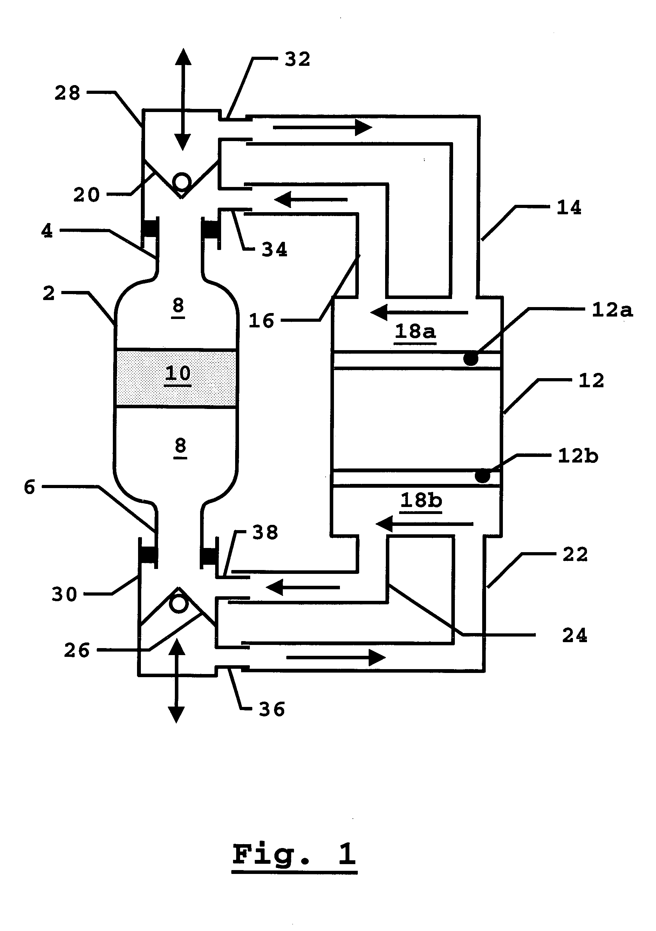

Considering now Fig. 1, an anaesthetic filter arrangement is shown comprising a filter housing 2 in which are formed openended conduit sections 4,6 for leading a gas to and from an interior gas-flow portion 8 of the housing 2. Contained within the interior portion 8 and arranged for gas communication with gas therein is a filter element 10 formed of a suitable sorption material for anaesthetic agent, such as zeolites of crystalline aluminium silicates which may be pellets or supported on a carrier; an activated carbon filter such as formed from carbon-impregnated material, carbon fibre cloth, or granulated or microporous carbon material; or other sorbtive microporous material.

A Peltier effect element 12 is provided with a warming surface 12a in intimate thermal contact with a first gas flow circuit 14,16 using, in the present embodiment, a first known heat exchange unit 18a, such as may simply be formed of gas flow conduit having a plurality of internal ridges, fins or channels to increase the surface area over which heat may be exchanged between gas from the first flow circuit 14,16 and the warming surface 12a. A cooling surface 12b, opposite the warming surface 12a of the Peltier element 12 is arranged in intimate thermal contact with a second gas flow circuit 22,24, here using a second known heat exchange unit 18b which together with the first heat exchange unit 18a may form a single known heat exchanger.

The two sections 14,16 of the first gas flow circuit are arranged in gas flow communication with the gas conduit 4 of the housing 2, each on an opposite flow side of a first one-way valve 20 arranged to permit a through flow of gas in a direction from the filter element 10 only.

The two sections 22,24 of the second gas flow circuit are arranged in gas flow communication with the gas conduit 6 of the housing 2, each on an opposite flow side of a second one-way valve 26 arranged to permit a through flow of gas in a direction from the filter element 10 only.

As is illustrated in Fig. 1, the one-way valves 20,26 may be provided integral with corresponding connector bodies 28,30 which are releasably connected to the conduit sections 4,6 respectively of the housing 2. These connector bodies 28,30 are intended for releasable coupling with a gas flow circuit of an anaesthetic ventilator system in a manner such that, in use, inspiration gas from the ventilator will flow into the housing 2 through the connector body 28 and expiration gas from a patient will flow into the housing 2 through the connector body 30. This enables the filter element 10 to be easily introduced into and removed from the gas flow circuit.

Gas ports 32,34 are provided either side of the first one-way valve 20 to provide gas communication between internal the connector body 28 and the two conduits 14,16 of the first gas flow circuit. Similarly, gas ports 36,38 are provided either side of the second one-way valve 26 to provide gas communication between internal the body 30 and the two conduits 22,24 of the second gas flow circuit.

The exemplary configuration of the anaesthetic filter arrangement of Fig. 1 is such that in use it is intended that gas from a patient (expiration gas) will pass through the filter element 10 in a direction from the conduit 6 to the conduit 4. Gas to be supplied to a patient (inspiration gas) is intended to pass through the filter element 10 in an opposite direction, that is, from the conduit 4 to the conduit 6. The intended directions of gas flow through the filter arrangement are shown by the arrows in Fig. 1.

In use, the operation and location of the second one-way valve 26 causes expiration gas to flow through the conduit 22 of the second gas flow circuit and to the second heat exchange unit 18b where it becomes cooled by the action of the appropriately energised Peltier device 12 using, in a known manner, a power supply unit (not shown). The cooled gas passes from the second heat exchange unit 18b, through the conduit 24 and into the filter housing 2 through the conduit section 6. The cooled gas then passes through the filter element 10, which retains anaesthetic agent present in the expiration gas, and out of the filter housing 2 through the conduit 4 and the first one-way valve 20. The amount of anaesthetic agent retained by the filter element 10 is enhanced because of the cooling effect of the gas passing through it.

As the expiration gas is cooled then water normally present in expiration gas may condense. It may be useful to provide a known water trap (not shown) in communication with the cooled expiration gas at a location before it contacts the filter element 10 to collect this condensate. More usefully such a water trap may be provided in communication also with inspiration gas at a location after it contacts the filter element 10 to moisten, in a known manner, the inspiration gas before delivery to a patient.

The operation and location of the first one-way valve 20 causes inspiration gas to pass through the conduit 14 of the first gas flow circuit and to the first heat exchange unit 18a where it becomes heated by the action of the appropriately energised Peltier device 12. The warmed gas passes from the first heat exchange unit 18a, through the conduit 16 the filter housing 2 through the conduit section 4. The warmed gas then passes through the filter element 10, which releases previously retained anaesthetic agent into the inspiration gas, and out of the filter housing 2 through the conduit 6 and the second one-way valve 26. The release of the anaesthetic agent by the element 10 is thus enhanced because of the warming effect of the inspiration gas passing through it.

Typically, a small amount of carbon dioxide that is present in the expiration gas will be retained by the filter element 10 and will be released into the inspiration gas together with the anaesthetic agent. This will be to the detriment of the anaesthetic ventilation therapy being provided. It has been discovered by the inventors that the amount of carbon dioxide which, in use, is re-supplied towards a patient from the filter arrangement according to the present invention is unexpectedly reduced in comparison to the amount re-supplied from a known filter arrangement.

As described above, the Peltier element 12 and the heat exchange units 18a,18b together form a thermal regulating device for varying the temperature of the filter element 10 by varying the temperature of the gases incident on the filter element 10. The temperature of the incident gases can be controlled by varying the electric current flowing through the Peltier element 12 which, in a known manner, varies the degree of cooling/heating produced by the element 12.

It will be appreciated that in the exemplary configuration of Fig. 1 the Peltier device 12 may be appropriately energised so that the warming side becomes 12a and the cooling side becomes 12b. In the use described above expiration gas will now become warmed to thereby diminish the retention by the filter element 10 of gaseous anaesthetic present in the expiration gas. The inspiration gas will become cooled to thereby diminish the release of previously retained anaesthetic in to the inspiration gas. This may be useful, for example, when trying to wake up a patient.

Moreover, the Peltier device 12 and heat exchange units 18a,18b may be substituted with other active thermal regulation devices, such as refrigerant compression, capable of providing a cooling and optionally a heating effect at the filter element 10. A passive heat exchanger may also substitute for the Peltier device 12 and heat exchange units 18a,18b. In such a passive heat exchanger the heat of the relatively warmer expiration gas is used to warm inspiration gas and itself becomes cooled in the process.

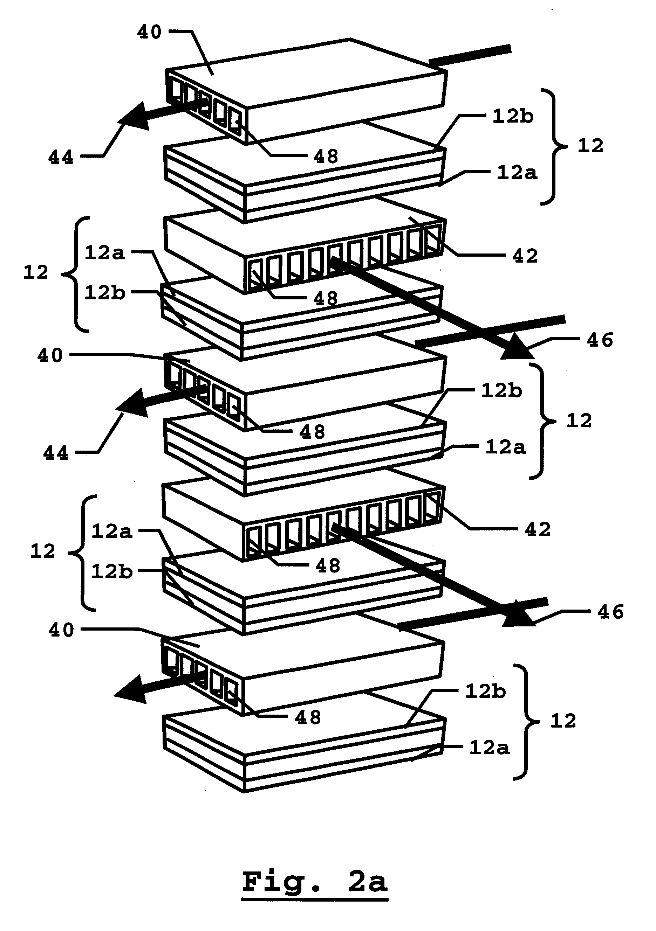

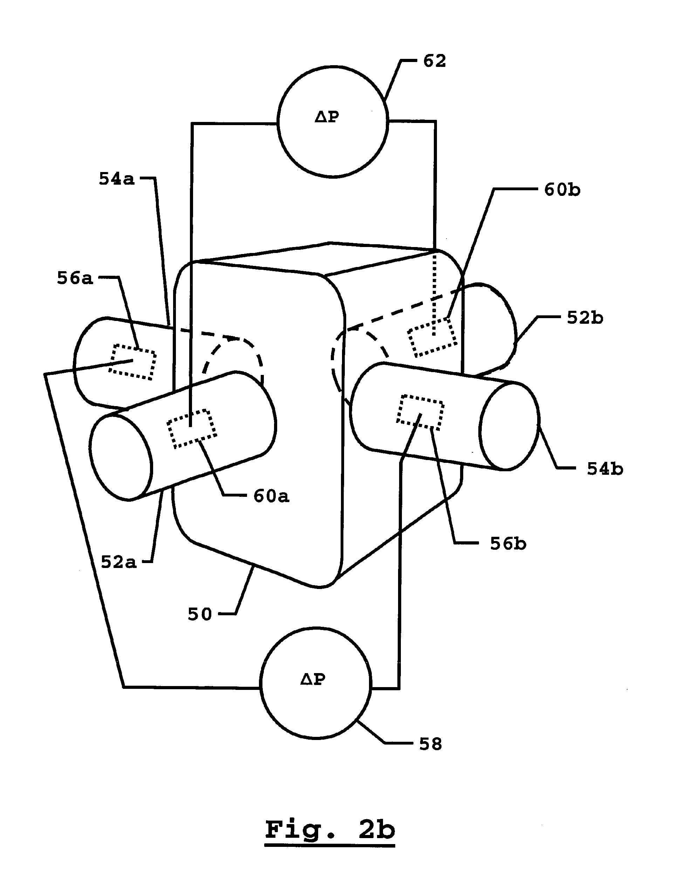

An example of a Peltier effect thermal regulating device suitable for use in, for example, the anaesthetic filter arrangement of Fig. 1 is shown in Figs. 2a and 2b. Considering now Fig. 2a, an internal portion of the device is shown in an exploded form to enable components to be more clearly seen. A plurality, here five, Peltier effect elements 12 are arranged in a stack so that, when the elements 12 are suitably energised, warming 12a and cooling 12b faces of adjacent elements face one another. Between each Peltier element 12 and in intimate thermal contact therewith is arranged a gas flow conduit 40,42. Each gas flow conduit is orientated to provide a direction of gas through-flow (indicated by arrows 44,46 in the Fig. 2) selected dependent on which of the faces 12a or 12b of the adjacent Peltier elements 12 it is in intimate thermal contact with. As illustrated in the present example, all of those gas flow conduits 40 that are arranged in thermal contact with a cooling side 12b of Peltier elements 12 are orientated to provide for through flow of gas in a single flow direction 44 that is orthogonal to a single flow direction 46 for gas flowing through those conduits 42 that are arranged in thermal contact with a warming side 12a of a Peltier element 12.

As is also illustrated in Fig. 2a, each conduit 40,42 is preferably formed with an increased internal surface area that in the present embodiment is realised by the inner surfaces of channels 48. The increased surface area increases the efficiency with which thermal energy can communicate between gas within the conduit 40,42 and the associated face 12b,12a of a Peltier element 12.

Considering now Fig 2b, a housing 50 is provided to contain the stacked arrangement of Fig. 2a. An orthogonal arrangement of externally accessible gas flow conduits 52a,b, 54a,b is provided in opposing walls of the housing 50 to define flow paths for gas between external and internal the housing 50 that communicate with respective flow paths 44,46 through the associated conduits 40,42 of the stack of Fig. 2a. Thus, referring to Fig. 1, it is intended that the flow conduits 22,24 of the second gas flow circuit will be connected with the gas flow conduits 52a,52b of the housing 50 and that the flow conduits 14,16 of the first gas flow circuit will be connected with the gas flow conduits 54a,54b of the housing 50.

Usefully, although not essentially, pressure sensors 56a,b may be located within the conduits 54a,b of the housing 50 to provide pressure readings to a differential pressure (ΔP) meter 58 by which gas flow (here inspiration gas flow) through the housing 48 can be calculated in a manner well known in the art. Similar pressure sensors 60a,b may be located within the conduits 52a,b and connected to a differential pressure meter (ΔP) 62 in order to gain a measure of gas flow (here expiration gas flow) through the housing 50.

## Claims

1. An anaesthetic filter arrangement comprising a filter housing (2) having an interior portion (8) arranged to provide an internal gas flow path for an inspiration gas and for an expiration gas passing through the housing (2); and a filter element (10) disposed in gas communication with the interior portion (8) for sorption of anaesthetic agent from the expiration gas and subsequent desorption of the anaesthetic agent into the inspiration gas;
**characterised in that** the arrangement further comprises thermal regulating means (12,18a,18b;12,40,42) operable to decrease thermal energy at the filter element (10) to vary one or both the sorption and the desorption of the anaesthetic agent.

2. An arrangement as claimed in Claim 1 **characterised in that** the thermal regulating means (12,18a,18b;12,40,42) is further operable to increase thermal energy at the filter element (10) to vary one or both the sorption and desorption of the anaesthetic agent.

3. An arrangement as claimed in Claim 2 **characterised in that** the thermal regulating means (12,18a,18b;12,40,42) is configured to alternately decrease and increase the thermal energy at the filter element (10) in synchronism with an alternate passing through the housing (2) of the expiration gas and of the inspiration gas respectively.

4. An arrangement as claimed in any preceding claim **characterised in that** the thermal regulating means (12,18a,18b;12,40,42) is configured to cool the expiration gas passing through the housing (2) to decrease the thermal energy at the filter element (10).

5. An arrangement as claimed in Claim 4 **characterised in that** the thermal regulating means (12,18a,18b;12,40,42) is further configured to warm the inspiration gas passing through the housing (2) to increase the thermal energy at the element (10).

6. An arrangement as claimed in Claim 4 or Claim 5 **characterised in that** the thermal regulating means (12,40,42) comprises a stackwise arrangement of a plurality of thermo-electric elements (12), each having a cooling surface (12b) and an opposing warming surface (12a) and arranged in the stack such that a same surface of each adjacent element is facing; and an externally accessible gas flow conduit (42;40) interposed between and in intimate thermal contact with the facing surfaces (12b;12a) of the adjacent elements (12).

7. An arrangement as claimed in Claim 6 **characterised in that** each gas flow conduit (42;40) of the stackwise arrangement is orientated to provide a gas flow path having a flow direction (46;44) depending on the facing surfaces (12a;12b) of the adjacent thermo-electric elements (12) it contacts.
